# EUROPEAN PATENT APPLICATION

(11) **EP 4 825 386 A1**
(43) Date of publication of application: **21.10.2026**
(21) Application number: 24890806.3
(22) Date of filing: 15.11.2024
(51) Int. Cl.: A61K 36/48, A61P 27/02

(54) **METHOD FOR IMPROVING DRY EYE SYNDROME USING ETHANOL EXTRACT OF SOYBEAN SEEDS AND COMBINATION THEREOF WITH ETHANOL DISTILLATE OF SOYBEAN SEEDS**

(30) Priority: 15.11.2023 US 202363599449 P
(71) Applicant: Botanicure CO., LTD., Tainan City 744092 (TW)
(72) Inventor: CHU, I-hung, Tainan City Taiwan 744092 (TW); WENG, Chih-yi, Tainan City Taiwan 744092 (TW)
(74) Representative: Mewburn Ellis LLP
(86) International application number: PCT/CN2024/132284
(87) International publication number: WO 2025/103454

(57) **Abstract**

Disclosed in the present invention are an ethanol extract of soybean seeds and a combination containing the ethanol extract of soybean seeds and an ethanol distillate, which can be used to improve dry eye syndrome.

## Description

### TECHNICAL FIELD

The present invention relates to a method for alleviating dry eye syndrome by using an ethanol extract of soybean seeds *(Glycine max* seed) and a combination of the ethanol extract and an ethanol-distillated product.

### BACKGROUND ART

Dry eye syndrome (DES) is mainly caused by insufficient tear secretion and excessive tear evaporation. The main symptoms of dry eye syndrome include dryness, redness, itching, photophobia and pain. In severe cases, dry eye syndrome may even cause visual disturbance and ocular surface damage.

Clinically, artificial tears are mainly used to treat mild dry eyes, while severe cases require anti-inflammatory drugs (SAIDs) for treatment. However, artificial tears and anti-inflammatory drugs can only temporarily alleviate eye discomfort and cannot increase tear secretion.

Soybean *(Glycine max)* is a plant of the genus *Glycine* in the family Fabaceae. The main part used is the seed, which is considered a good source of protein intake. In TW I640318 B (corresponding to US 10543243 B2), the applicant disclosed an extraction composition, which includes an extraction product obtained by extracting soybean seeds using water or ethanol below 90 wt%, and a distillation product obtained by distilling soybean seeds using water or ethanol below 15 wt%. The extraction composition has been shown to have the effect of promoting skin wound healing and nerve cell proliferation, and treating dementia and breast cancer. In TW I724417 B (corresponding to US 11083766 B2), the applicant disclosed the use of the extraction composition in alleviating pain and skin inflammation caused by cancer radiotherapy.

As far as the applicant knows, there is no document or patent case that has ever disclosed that the extract or distillation product of soybean seeds can be used to alleviate dry eye syndrome.

### SUMMARY OF INVENTION

In the present invention, the applicant has discovered through experiments that the use of an ethanol extract of soybean seeds *(Glycine max* seed) alone and combination thereof with an ethanol-distillated product can effectively increase the tear secretion of mice with scopolamine-induced dry eye syndrome, and is believed to have the effect of alleviating dry eye syndrome.

Therefore, in a first aspect, the present invention provides a method for alleviating dry eye syndrome, which comprises administering a pharmaceutical product comprising the ethanol extract of soybean seeds as described above to a subject in need thereof.

In a second aspect, the present invention provides a method for alleviating dry eye syndrome, which comprises administering a pharmaceutical product comprising the combination of the ethanol extract of soybean seeds and the ethanol-distillated product as described above to a subject in need thereof.

In a third aspect, the present invention provides an ethanol extract of soybean seeds for use in preparing a pharmaceutical product for alleviating dry eye syndrome.

In a fourth aspect, the present invention provides the combination as described above for use in preparing a pharmaceutical product for alleviating dry eye syndrome.

In a fifth aspect, the present invention provides a pharmaceutical product for alleviating dry eye syndrome which comprises the ethanol extract of soybean seeds as described above.

In a sixth aspect, the present invention provides a pharmaceutical product for alleviating dry eye syndrome which comprises the combination as described above.

Preferably, the ethanol extract is obtained by subjecting the soybean seeds to extraction using 70 wt% to 90 wt% ethanol.

Preferably, the ethanol-distillated product is obtained by subjecting soybean seeds to distillation using 0.01 wt% to 15 wt% ethanol.

Preferably, the ethanol-distillated product is obtained by subjecting soybean seeds to distillation using 0.13 wt% to 15 wt% ethanol.

Preferably, the combination includes 0.05 wt% to 1 wt% of the ethanol extract and 70 wt% to 90 wt% of the ethanol-distillated product.

Preferably, the pharmaceutical product further includes a pharmaceutically acceptable carrier.

Preferably, the pharmaceutical product is in a dosage form for oral administration, topical administration or parenteral administration.

### BRIEF DESCRIPTION OF THE DRAWINGS

The above and other objects, features and advantages of the present invention will become apparent after referring to the following detailed description and preferred embodiments and the accompanying drawings, in which:
FIG. 1 shows the wetting length measured for each group of mice in Example 1, wherein "*" and "**" indicate *p*<0.05 and *p*<0.01, respectively, when compared with the pathological control group.
FIG. 2 shows the wetting length measured for each group of mice in Example 2, wherein "**" indicates *p*<0.01 when compared with pathological control groups 1 to 3.

### DETAILED DESCRIPTION

For the purposes of this specification, it will be expressly understood that the word "comprising" means "including, but not limited to," and that the word "comprises" has a corresponding meaning.

The present invention provides an ethanol extract of soybean seeds *(Glycine max* seed) for use in preparing a pharmaceutical product for alleviating dry eye syndrome. The present invention also provides a combination of an ethanol extract of soybean seeds and an ethanol-distillated product for use in preparing a pharmaceutical product for alleviating dry eye syndrome.

In addition, the present invention provides a pharmaceutical product for alleviating dry eye syndrome, and the pharmaceutical product includes the ethanol extract of soybean seeds as described above. The present invention also provides a pharmaceutical product for alleviating dry eye syndrome, and the pharmaceutical product includes the combination as described above.

As used herein, the terms "dry eye syndrome," "xerophthalmia," "sclerophthalmia," "keratoconjunctivitis sicca," and "dysfunctional tear syndrome" are used interchangeably and are intended to cover at least one of the following forms: aqueous tear-deficient dry eye, mucin-deficient dry eye, lipid-deficient dry eye, and evaporative dry eye.

Dry eye syndrome to which the present invention is applicable may be caused by a variety of factors, including, but not limited to: a deficiency in tear-flow system; sleep disorders, such as insomnia and sleep apnea syndrome; natural aging process, particularly menopause; side effects of medications (e.g., antidepressants, blood pressure medications, Parkinson's disease medications, antihistamines and birth control pills); diseases affecting tear production capability, such as Sjogren's syndrome, rheumatoid arthritis, and collagen vascular disease; wearing of contact lens; smoking; particulate matter (PM), such as PM 2.5; dry climates; insufficient blinking; and structural problems that prevent the eyelid from closing properly.

As used herein, the terms "soybean (*Glycine max),"* "edamame *(Glycine max),"* "yellow soybean *(Glycine max),"* and "soybean" may be used interchangeably and are intended to cover soybeans that are readily available to one skilled in the art or collected from natural sources.

According to the present invention, the methods for ethanol extraction and ethanol distillation of soybean seeds can be carried out by using the techniques well known and commonly used by those skilled in the art. In this regard, reference can be made to, for example, TW I724417 B (corresponding to US 11083766 B2) and TW I640318 B (corresponding to US 11452753 B2, US 11400128 B2 and US 10543243 B2).

It is understood that the operating conditions of the methods for the ethanol extraction and the ethanol distillation of soybean seeds will be further varied depending on factors such as the processing method of the soybean seeds and the ratio of the soybean seeds to ethanol, so as to achieve the best extraction effect. The selection of these operating conditions can be routinely determined by those skilled in the art.

According to the present invention, the ethanol extraction and the ethanol distillation of soybean seeds can be performed using fresh soybean seeds, or can be performed using soybean seeds that have been previously processed by a process selected from the group consisting of: drying, grinding, chopping, pulverizing, and combinations thereof.

According to the present invention, the ethanol extraction and the ethanol distillation can be performed using soybean seeds and an ethanol solution in a weight ratio of 1:1 to 1:100, respectively. In a preferred embodiment of the present invention, the weight ratio is 1:10.

According to the present invention, the ethanol extraction can be performed by using 70 wt% to 90 wt% ethanol, preferably, 70 wt% to 80 wt% ethanol. In a preferred embodiment of the present invention, the ethanol extraction is performed by using 70 wt% ethanol.

According to the present invention, the ethanol extraction can be performed at a temperature ranging from 22°C to 45°C. In a preferred embodiment of the present invention, the temperature is 45°C.

According to the present invention, the ethanol distillation can be performed by using 0.01 wt% to 15 wt% ethanol, preferably, 0.13 wt% to 15wt % ethanol. In a preferred embodiment of the present invention, the ethanol distillation is performed by using 0.13 wt% ethanol.

According to the present invention, the ethanol distillation can be carried out at a temperature ranging from 55°C to 98°C. In a preferred embodiment of the present invention, the temperature is 90°C.

According to the present invention, the combination may include 0.05 wt% to 5 wt% of the ethanol extract of soybean seeds and 20 wt% to 90 wt% of the ethanol-distillated product of soybean seeds, preferably, 0.05 wt% to 1 wt% of the ethanol extract of soybean seeds and 70 wt% to 90 wt% of the ethanol-distillated product of soybean seeds, and more preferably, 0.05 wt% to 0.3 wt% of the ethanol extract of soybean seeds and 70 wt% to 90 wt% of the ethanol-distillated product of soybean seeds.

According to the present invention, the pharmaceutical product may be in a dosage form suitable for parenteral administration, oral administration or topical administration.

According to the present invention, the pharmaceutical product may further include a pharmaceutically acceptable carrier widely used in drug manufacturing technology. For example, the pharmaceutically acceptable carrier may include one or more agents selected from the following: solvent, buffer, emulsifier, suspending agent, decomposer, disintegrating agent, dispersing agent, binding agent, excipient, stabilizer, chelating agent, diluent, gelling agent, preservative, wetting agent, lubricant, absorption delaying agent, liposome, thickeners and the like. The selection and amount of these reagents are within the professional knowledge and routine skills of those familiar with this technology.

According to the present invention, the pharmaceutical product can be manufactured into a dosage form suitable for parenteral administration (including injections, for example, a sterile aqueous solution or dispersion) using techniques well known to those skilled in the art, and administered by a route selected from the group consisting of: intraperitoneal injection, intrapleural injection, intramuscular injection, intravenous injection, intraarterial injection, intraarticular injection, intrasynovial injection, intrathecal injection, intracranial injection, intraepidermal injection, subcutaneous injection, intradermal injection, intralesional injection, and sublingual administration.

According to the present invention, the pharmaceutical product can be manufactured into a dosage form suitable for oral administration using techniques well known to those skilled in the art, including, but not limited to: sterile powders, tablet, troche, lozenge, pellet, capsule, dispersible powder or granule, solution, suspension, emulsion, syrup, elixir, slurry and the like.

According to the present invention, the pharmaceutical product can be manufactured into an external preparation suitable for topical application to the skin using techniques well known to those skilled in the art, including, but not limited to: emulsion, gel, ointment, cream, patch, liniment, powder, aerosol, spray, lotion, serum, paste, foam, drop, suspension, salve and bandage.

According to the present invention, the external preparation is prepared by mixing the pharmaceutical product of the present invention with a base well known to those skilled in the art.

According to the present invention, the base may contain one or more additives selected from the following: water, alcohols, glycols, hydrocarbons (such as petroleum jelly and white petrolatum), waxes (such as paraffin and yellow wax), preserving agents, antioxidants, surfactants, absorption enhancers, stabilizers, gelling agents (such as Carbopol^{®} 941, microcrystalline cellulose, and carboxymethylcellulose), active agents, humectants, odor absorbers, fragrances, pH adjusting agents, chelating agents, emulsifiers, occlusive agents, emollients, solubilizing agents, penetration enhancers, thickeners, anti-irritants, preservatives, colorants and propellants, etc. The selection and amount of these additives are within the professional qualities and routine skills of those familiar with the technology.

More preferably, the pharmaceutical product is an ointment including 0 wt% to 10.15 wt% of a thickener, 0 wt% to 13.75 wt% of an emulsifier, and 0.28 wt% to 0.35 wt% of a preservative.

In a preferred embodiment of the present invention, the thickener is polyacrylic acid (carbomer), glyceryl polyacrylate and hyaluronic acid.

According to the present invention, the emulsifier may be selected from the group consisting of: hydrogenated polydecene (Parleam), borneol, propylene glycol, polysorbate 60, trideceth-6 (Mihacol 139), isoceteth-20 (Mihacol 250), stearic acid, stearyl alcohol, polysorbate 80 and potassium hydroxide.

In a preferred embodiment of the present invention, the preservative is benzyl alcohol or chloroxylenol.

In another aspect, the present invention provides a method for alleviating dry eye syndrome, which comprises administering a pharmaceutical product including the above-mentioned ethanol extract of soybean seed to a subject in need thereof.

The present invention also provides a method for alleviating dry eye syndrome, which comprises administering a pharmaceutical product including the combination as described above to a subject in need thereof.

As used herein, the terms "administrating" and "administration" may be used interchangeably, and refer to introducing, providing or delivering a predetermined active ingredient to a subject by any appropriate route to perform its intended effect.

As used herein, the term "subject" means any mammal of interest, such as humans, monkeys, cows, sheep, horses, pigs, goats, dogs, cats, mice, and rats.

According to the present invention, the dosage and frequency of administration of the ethanol extract of soybean seeds and the combination will vary depending on the following factors: the severity of the disease to be treated, the route of administration, and the age, physical condition and response of the individual to be treated. In General, the ethanol extract of soybean seeds and the combination may be administered in the form of a single dose or divided into several doses. For example, the ethanol extract of soybean seeds and the combination may be administered twice a day.

The present invention will be further described with reference to the following examples. However, it should be understood that these examples are only for illustration and should not be construed as limitations on the implementation of the present invention.

### <Examples>

### General experimental materials:

### 1. Preparation of ethanol extract and ethanol-distillated product of soybean seeds (Glycine max seeds):

The ethanol extract and ethanol-distillated product of soybean seeds used in this example were prepared by referring to the method described in the example of TW I640318 B (corresponding to US 10543243 B2).

Briefly, soybean seed powder collected from Tainan City was mixed with 70 wt% ethanol in a weight ratio of 1:10, followed by heating extraction at 45°C. Thereafter, the resulting mixture was filtered to remove solids, and vacuum filtered and dried to obtain an ethanol extract of soybean seeds.

In addition, soybean seed powder was mixed with 0.13 wt% ethanol in a weight ratio of 1:10, followed by distillation at 90°C and collection of a condensate, thereby obtaining an ethanol-distillated product of soybean seeds.

### 2. Experimental animals:

Male C57BL/6 mice (weighing approximately 24 g) used in this example were purchased from BioLasco Taiwan Co., Ltd. All experimental animals were kept in an animal room with 12 hours of light and 12 hours of darkness, maintained at 24-26°C and a relative humidity of 55%-65%, with *ad libitum* access to water and feed. The handling of experimental animals and all experimental programming complied with the experimental animal management standards of the Association for Assessment and Accreditation of Laboratory Animal Care, International.

### General experimental methods:

### 1. Statistical analysis:

The experimental data obtained were expressed as "mean ± standard error of the mean (SEM)" and analyzed by Student's t-test to evaluate the differences between the groups. If the statistical analysis result obtained was p *<* 0.05, it indicated statistical significance.

### Example 1. Evaluation of the efficacy of ethanol extract of soybean seeds in alleviating dry eye syndrome

### Experimental Materials:

### 1. Preparation of ointment containing ethanol extract of soybean seeds:

Ointments 1 and 2 used in this example had the formulations shown in Table 1 below. These two ointments were prepared according to the following steps: the ethanol extract of soybean seeds was dissolved in water and heated to a temperature ranging from 95°C to 100°C, and then adding polyacrylic acid (carbomer), glyceryl polyacrylate, and hyaluronic acid with stirring, followed by adding benzyl alcohol and chloroxylenol after cooling the resulting mixture.

**Table 1. Formulations of Ointments 1 and 2**

| | Ointment 1 | Ointment 2 |
|---|---|---|
| Ingredient | Content (wt%) | |
| Ethanol extract of soybean seeds | 0.1 | 1 |
| Polyacrylic acid | 0.15 | 0.15 |
| Glyceryl polyacrylate | 9.99 | 9.99 |
| Hyaluronic acid | 0.01 | 0.01 |
| Benzyl alcohol | 0.28 | 0.28 |
| Chloroxylenol | 0.07 | 0.07 |
| Water | 89.4 | 88.5 |

### Experimental methods:

### A. Induction dry eye syndrome and administration:

First, the mice (14 weeks old) were randomly divided into 4 groups, including a normal control group (n=6), a pathological control group (n=6), experimental group 1 (n=6) and experimental group 2 (n=5). Then, the mice in the pathological control group, the comparative group and the experimental groups 1 and 2 were subjected to subcutaneous injections of scopolamine (Sigma, Cat. No. S0929) (at a dose of 0.5 mg/mouse, prepared in 0.2 mL of normal saline) twice a day to induce dry eye syndrome. The mice in the normal control group did not receive any treatment.

On the first day after the start of scopolamine injections, Ointments 1 and 2 were topically applied to the skin around the eyes of the mice in experimental groups 1 and 2, respectively (at a dose of 50 mg of ointment per 1 cm² of area), twice daily for a total of 21 days. The mice in the normal control group and the pathological control group did not receive any treatment.

### Results:

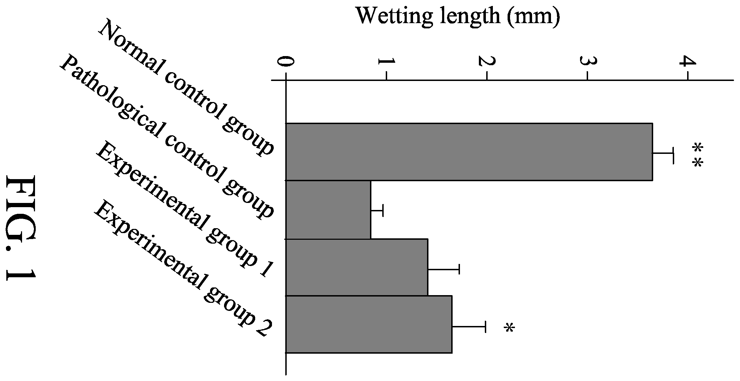

FIG. 1 showed the wetting lengths measured for each group of the mice. As shown in FIG. 1, the wetting length of the pathological control group was significantly lower than that of the normal control group, indicating that the scopolamine successfully induced dry eye syndrome in the mice and resulted in insufficient tear secretion. Compared with the pathological control group, the wetting lengths of experimental groups 1 and 2 were both markedly increased, and became more pronounced as the concentration of the ethanol extract of soybean seeds increased. In particular, the wetting length of experimental group 2 was significantly higher than that of the pathological control group.

These experimental results showed that both Ointments 1 and 2 can effectively increase tear secretion. Therefore, the ethanol extract of soybean seeds can alleviate dry eye syndrome in a dose-related manner. Thus, the ethanol extract of soybean seeds was considered to have high potential for use in preparation of a pharmaceutical product for alleviating dry eye syndrome.

Additionally, the applicant also prepared an ointment containing 1 wt% of the ethanol extract of soybean seeds and 90 wt% of the ethanol-distillated product of soybean seeds according to the aforementioned method, and conducted the same test. The results showed that the wetting length measured was significantly higher than those of Ointments 1 and 2 (data not shown). Therefore, the applicant further tested the efficacy of a combination of the ethanol extract of soybean seeds and the ethanol-distillated product of soybean seeds in the following examples.

### Example 2. Evaluation of the efficacy of a combination of the ethanol extract of soybean seeds and the ethanol-distillated product thereof in alleviating dry eye syndrome

### Experimental Materials:

### 1. Preparation of ointment containing the ethanol extract of soybean seeds and the ethanol-distillated product:

Ointments 3 to 5 used in this example had the formulations shown in Table 2 below. These three ointments were prepared generally according to the method described in Example 1, with the differences being: the ethanol extract of soybean seeds was dissolved in water together with the ethanol-distillated product; in Ointment 4, polyacrylic acid, glyceryl polyacrylate, and hyaluronic acid were replaced with hydrogenated polydecene (Parleam), borneol, propylene glycol, polysorbate 60, trideceth-6 (Mihacol 139), and isoceteth-20 (Mihacol 250); and in Ointment 5, polyacrylic acid, glyceryl polyacrylate, and hyaluronic acid were replaced with borneol, stearic acid, stearyl alcohol, polysorbate 80, and potassium hydroxide, which were added after heating.

**Table 2. Formulations of Ointments 3 to 5**

| | Ointment 3 | Ointment 4 | Ointment 5 |
|---|---|---|---|
| Ingredient | Content (wt%) | | |
| Ethanol extract of soybean seeds | 0.05 | 0.3 | 0.3 |
| Ethanol-distillated product of soybean seeds | 70 | 90 | 70 |
| Polyacrylic acid | 0.14 | - | - |
| Glyceryl polyacrylate | 10 | - | - |
| Hyaluronic acid | 0.01 | - | - |
| Hydrogenated polydecene | - | 5 | - |
| Borneol | - | 0.3 | 0.3 |
| Propylene glycol | - | 1 | - |
| Polysorbate 60 | - | 0.1 | - |
| Trideceth-6 | - | 1.2 | - |
| Isoceteth-20 | - | 0.5 | - |
| Stearic acid | - | - | 6 |
| Stearyl alcohol | - | - | 5 |
| Polysorbate 80 | - | - | 2.35 |
| Potassium hydroxide | - | - | 0.1 |
| Benzyl alcohol | 0.28 | 0.35 | 0.35 |
| Chloroxylenol | 0.07 | - | - |
| Water | 19.45 | 1.25 | 15.6 |

### Experimental methods:

### A. Induction of dry eye syndrome and administration:

First, the mice (8 weeks old) were randomly divided into nine groups, including three normal control groups (i.e., normal control groups 1 to 3, n=6 per group), three pathological control groups (i.e., pathological control groups 1 to 3, n=6 per group), and three experimental groups (i.e., experimental groups 1 to 3, n=7 per group). Subsequently, scopolamine was injected into the mice of the pathological control groups 1 to 3 and the experimental groups 1 to 3 four times daily, according to the method described in Example 1, and the mice were exposed to a dry environment with airflow generated by fans for a total of 7 days to induce dry eye syndrome. The mice in the normal control groups 1 to 3 did not receive any treatment.

On the 8^{th} day after the start of scopolamine injections, Ointments 3 to 5 were topically applied to the skin around the eyes of the mice in the experimental groups 1 to 3, respectively (at a dose of 50 mg of ointment per 1 cm² of area), twice daily for a total of 7 days. The mice in the normal control groups 1 to 3 and the pathological control groups 1 to 3 did not receive any treatment.

### B. Schirmer's Tear Test

On the 14^{th} day after the start of the scopolamine injections, the wetting length of each group of the mice was measured and recorded by referring to the method described in Example 1.

### Results:

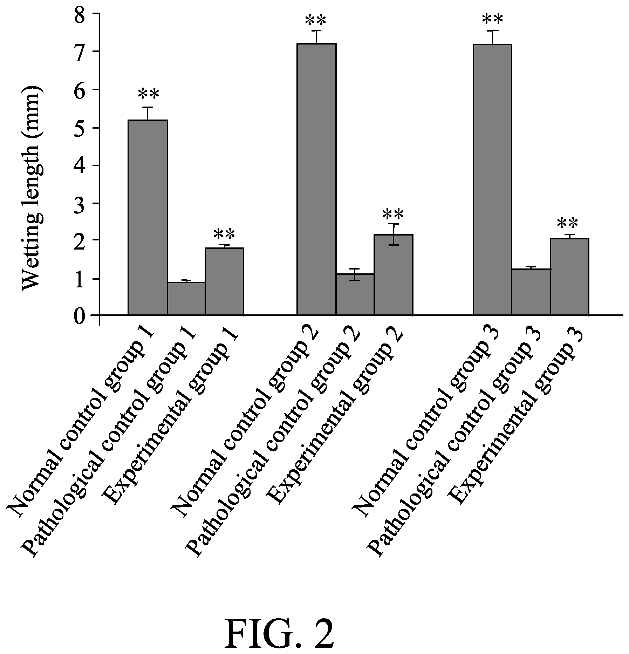

FIG. 2 shows the measured wetting length of each group of mice. As seen in FIG. 2, the wetting lengths of the pathological control groups 1 to 3 were significantly lower than those of the normal control groups 1 to 3, respectively, indicating that scopolamine and the dry environment successfully induced dry eye syndrome and caused insufficient tear secretion in the mice. Compared with the pathological control groups 1 to 3, the wetting lengths of the experimental groups 1 to 3 were significantly increased. These experimental results show that Ointments 3 to 5 can all effectively increase tear secretion. Therefore, the combination of the ethanol extract of soybean seeds and the ethanol distillation product is considered to have high potential for use in preparation of a pharmaceutical product for alleviating dry eye syndrome.

All patents and literatures cited in this specification are incorporated herein by reference in their entirety. In case of conflict, the detailed description of the present case (including definitions) shall prevail.

Although the present invention has been described with reference to the specific embodiments mentioned above, it is apparent that many modifications and variations can be made without departing from the scope and spirit of the present invention. Therefore, it is intended that the present invention be limited only as indicated by the appended claims.

## Claims

1. Use of an ethanol extract of soybean seeds for preparation of a pharmaceutical product for alleviating dry eye syndrome.

2. Use of a combination including an ethanol extract of soybean seeds and an ethanol-distillated product for preparation of a pharmaceutical product for alleviating dry eye syndrome.

3. The use according to Claim 1 or 2, **characterized in that** the ethanol extract is obtained by subjecting the soybean seeds to extraction using 70 wt% to 90 wt% ethanol.

4. The use according to Claim 2, **characterized in that** the ethanol-distillated product is obtained by subjecting soybean seeds to distillation using 0.01 wt% to 15 wt% ethanol.

5. The use according to Claim 2, **characterized in that** the ethanol-distillated product is obtained by subjecting soybean seeds to distillation using 0.13 wt% to 15 wt% ethanol.

6. The use according to Claim 2, **characterized in that** the combination includes 0.05 wt% to 1 wt% of the ethanol extract and 70 wt% to 90 wt% of the ethanol-distillated product.

7. The use according to Claim 1 or 2, **characterized in that** the pharmaceutical product further includes a pharmaceutically acceptable carrier.

8. The use according to Claim 1 or 2, **characterized in that** the pharmaceutical product is in a dosage form for oral administration, topical administration, or parenteral administration.

9. A method for alleviating dry eye syndrome, **characterized in that** the method comprises: administering to a subject in need thereof a pharmaceutical product, the pharmaceutical product comprising an ethanol extract of soybean seeds.

10. A method for alleviating dry eye syndrome, **characterized in that** the method comprises: administering to a subject in need thereof a pharmaceutical product, the pharmaceutical product comprising a combination which includes an ethanol extract of soybean seeds and an ethanol-distillated product.

11. The method according to Claim 9 or 10, **characterized in that** the ethanol extract is obtained by subjecting the soybean seeds to extraction using 70 wt% to 90 wt% ethanol.

12. The method according to Claim 10, **characterized in that** the ethanol-distillated product is obtained by subjecting soybean seeds to distillation using 0.01 wt% to 15 wt% ethanol.

13. The method according to Claim 10, **characterized in that** the ethanol-distillated product is obtained by subjecting soybean seeds to distillation using 0.13 wt% to 15 wt% ethanol.

14. The method according to Claim 10, **characterized in that** the combination includes 0.05 wt% to 1 wt% of the ethanol extract and 70 wt% to 90 wt% of the ethanol-distillated product.

15. The method according to Claim 9 or 10, **characterized in that** the pharmaceutical product further includes a pharmaceutically acceptable carrier.

16. The method according to Claim 9 or 10, **characterized in that** the pharmaceutical product is in a dosage form for oral administration, topical administration, or parenteral administration.

17. A pharmaceutical product for alleviating dry eye syndrome, **characterized in that** the pharmaceutical product comprises an ethanol extract of soybean seeds.

18. A pharmaceutical product for alleviating dry eye syndrome, **characterized in that** the pharmaceutical product comprises a combination which includes an ethanol extract of soybean seeds and an ethanol-distillated product.

19. The pharmaceutical product according to Claim 17 or 18, **characterized in that** the ethanol extract is obtained by subjecting the soybean seeds to extraction using 70 wt% to 90 wt% ethanol.

20. The pharmaceutical product according to Claim 18, **characterized in that** the ethanol-distillated product is obtained by subjecting soybean seeds to distillation using 0.01 wt% to 15 wt% ethanol.

21. The pharmaceutical product according to Claim 18, **characterized in that** the ethanol-distillated product is obtained by subjecting soybean seeds to distillation using 0.13 wt% to 15 wt% ethanol.

22. The pharmaceutical product according to Claim 18, **characterized in that** the combination includes 0.05 wt% to 1 wt% of the ethanol extract and 70 wt% to 90 wt% of the ethanol-distillated product.

23. The pharmaceutical product according to Claim 17 or 18, **characterized in that** the pharmaceutical product further includes a pharmaceutically acceptable carrier.

24. The pharmaceutical product according to Claim 17 or 18, **characterized in that** the pharmaceutical product is in a dosage form for oral administration, topical administration, or parenteral administration.
